(19) Europäisches Patentamt
European Patent Office
Office européen des brevets

(11) Veröffentlichungsnummer: **0 239 907 B1**

(12) # EUROPÄISCHE PATENTSCHRIFT

(45) Veröffentlichungstag der Patentschrift: **18.09.91**

(51) Int. Cl.[5]: **C07C 311/02**, C07C 311/13, A61K 31/19

(21) Anmeldenummer: **87104281.8**

(22) Anmeldetag: **24.03.87**

Die Akte enthält technische Angaben, die nach dem Eingang der Anmeldung eingereicht wurden und die nicht in dieser Patentschrift enthalten sind.

(54) **Neue Phenoxyalkylcarbonsäure-Derivate, Verfahren zu ihrer Herstellung, sowie diese Verbindungen enthaltende Arzneimittel.**

(30) Priorität: **29.03.86 DE 3610643**

(43) Veröffentlichungstag der Anmeldung:
**07.10.87 Patentblatt 87/41**

(45) Bekanntmachung des Hinweises auf die Patenterteilung:
**18.09.91 Patentblatt 91/38**

(84) Benannte Vertragsstaaten:
**AT BE CH DE ES FR GB GR IT LI LU NL SE**

(56) Entgegenhaltungen:
**EP-A- 0 004 011**
**EP-A- 0 223 593**

(73) Patentinhaber: **BOEHRINGER MANNHEIM GMBH**
**Sandhofer Strasse 116**
**W-6800 Mannheim 31(DE)**

(72) Erfinder: **Pill, Johannes, Dr. rer. nat. Dr. med.**
**In der Keitgasse 6**
**W-6906 Leimen 3(DE)**
Erfinder: **Wolff, Hans Peter., Dr. rer. nat.**
**Rebenweg 24**
**W-6945 Hirschberg-Grosssachsen(DE)**
Erfinder: **Witte, Ernst-Christian, Dr. rer. nat.**
**Beethovenstrasse 2**
**W-6800 Mannheim 1(DE)**
Erfinder: **Stegmeier, Karlheinz, Dr. rer. nat.**
**Kirchbergstrasse 17**
**W-6148 Heppenheim(DE)**

**EP 0 239 907 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft am Phenyl in 2- und 3-Stellung substituierte Phenoxyalkylcarbonsäuren und ihre Derivate, Verfahren zu ihrer Herstellung sowie Arzneimittel, die diese Verbindungen enthalten.

In der EP-A-0 004 011 sind Phenoxyalkylcarbonsäuren mit lipidsenkender und die Thrombozytenaggregation hemmender Wirkung beschrieben, die in 4-Stellung substituiert sind.

In der nicht vorveröffentlichten EP-A-0 223 593 sind Phenoxyessigsäure-Derivate beschrieben, die am Phenylring in meta-Stellung durch den Rest $R_2$-$SO_2$-NH-$(CH_2)_n$- substituiert sind. Hierin bedeutet $R_2$ Naphthyl, ß-Styryl, Phenyl$(CH_2)_p$, p die Zahl 0, 1, 2 oder 3 und n die Zahl 1, 2 oder 3, wobei Phenyl ein- oder mehrfach durch Halogen, $C_1$-$C_4$-Alkyl, $C_1$-$C_4$-Alkoxy, Hydroxyl, Trifluormethyl, Nitro oder Amino substituiert sein kann.

Es wurde nun überraschenderweise gefunden, daß auch die 2- bzw. 3-substituierten Phenoxyalkylcarbonsäure-Derivate eine lipidsenkende und thromboxan-$A_2$-antagonistische Wirkung zeigen.

Die vorliegende Erfindung betrifft daher neue Sulfonylphenylalkylamine der allgemeinen Formel I

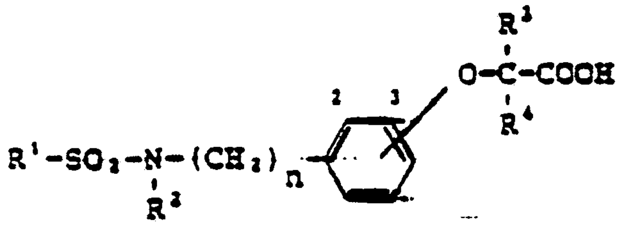

(I)

in welcher die Sulfonylaminoalkylgruppe in ortho- oder in meta-Stellung zur Phenoxyalkylcarbonsäure-Gruppe steht und in welcher

$R_1$ eine Aryl-, Aralkyl- oder eine Aralkenylgruppe bedeutet, deren Arylrest jeweils gegebenenfalls ein- oder mehrfach durch Halogen, Cyan, Alkyl, Trifluormethyl, Alkoxy, Hydroxyl, Nitro, Amino, Alkylamino, Dialkylamino, Acylamino, Acyl, Azido oder gegebenenfalls durch Halogen, Cyan, Alkyl, Trifluormethyl oder Alkoxy substituiertes Phenyl substituiert sein kann,

$R_2$ Wasserstoff, eine Alkyl- , Aralkyl- oder eine Acylgruppe darstellt,

$R_3$ und $R_4$, die gleich oder verschieden sein können, Wasserstoff oder eine niedere Alkylgruppe und

n die Zahlen 1 - 3

bedeuten, sowie deren physiologisch unbedenkliche Salze, Ester und Amide

mit der Maßgabe, daß die Phenoxyalkylcarbonsäure-Gruppe sowie deren physiologisch unbedenkliche Salze und $C_{1-4}$-Alkylester nicht in meta-Stellung stehen darf, wenn

$R_1$ Naphthyl, ß-Styryl, Phenyl$(CH_2)_p$, p die Zahl 0, 1, 2 oder 3, wobei Phenyl ein- oder mehrfach durch Halogen, $C_1$-$C_4$-Alkyl, $C_1$-$C_4$-Alkoxy, Hydroxyl, Trifluormethyl, Nitro oder Amino substituiert sein kann,

n die Zahl 1, 2 oder 3

$R_2$ Wasserstoff und $R_3$ und $R_4$ Wasserstoff

bedeuten,

jedoch 3-[2-(4-Chlor-phenylsulfonylamino)ethyl]phenoxyessigsäure sowie deren physiologisch unbedenkliche Salze, Ester und Amide nicht ausgenommen sind.

Besonders bevorzugt sind Verbindungen der Formel I, in der

$R_1$ eine Phenyl- oder Biphenylgruppe bedeutet, die jeweils gegebenenfalls durch Chlor, Brom, Cyan, Methyl, Trifluormethyl und Methoxy substituiert sein kann,

$R_2$ eine Methyl-, Octyl-, Benzyl-, Acetyl-, n-Octanoyl- und n-Hexadecanoylgruppe darstellt,

$R_3$ und $R_4$ Wasserstoff und

n die Zahl 2 bedeuten,

sowie deren physiologisch unbedenkliche Salze, Ester und Amide.

Die neuen Verbindungen der allgemeinen Formel I zeigen eine ausgezeichnete antagonistische Wirkung gegenüber Thromboxan $A_2$ sowie gegen Prostaglandin-endoperoxide. Sie inhibieren die Aggregation von Blutplättchen und verhindern die Konstriktion der glatten Muskulatur sowie die Bronchokonstriktion.

Diese Eigenschaften machen sie zu wertvollen Heilmitteln zur Behandlung z.B. von cardiovaskulären Erkrankungen und von Asthma und zur Prophylaxe einer Schocklunge. Sie können weiterhin verwendet werden bei Organtransplantationen und Nierendialyse und sind geeignet, Rezidive bei Magengeschwüren

zu verhindern Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können z.B. gegen cerebrale ischämische Zustände eingesetzt werden.

Zusätzlich vermögen sie den Acetateinbau in Cholesterin zu hemmen und eignen sich daher auch zur Behandlung von Fettstoffwelchselerkrankungen.

Als "Arylrest", allein oder in Verbindung mit einer Alkyl-oder Alkylenkette, sind in allen Fällen aromatische Kohlenwasserstoffe mit 6-14 C-Atomen, insbesondere der Phenyl-, der Biphenylyl-, der Naphthyl- und der Fluorenylrest zu verstehen. Diese Arylreste können in allen möglichen Positionen ein- oder mehrfach substituiert sein, wobei als Substituenten Halogen, $C_1$-$C_6$ Alkyl, $C_1$-$C_6$ Alkoxy, Hydroxyl, Trifluormethyl, Cyan, Nitro, Amino, $C_1$-$C_6$ Alkylamino, $C_1$-$C_{12}$ Dialkylamino, $C_1$-$C_6$ Acylamino, $C_1$-$C_6$ Acyl und Azid in Frage kommen. Bevorzugt ist der Phenylrest, der durch Halogen, bevorzugt Chlor und Brom, Cyan, Methyl, Trifluormethyl, 4-Chlorphenyl und Acetyl substituiert sein kann

Unter den Alkyl- und Alkoxysubstituenten der Aryl-, Aralkyl-und Aralkenylreste sind Reste mit 1-4 C-Atomen bevorzugt, insbesondere die Methyl-, Ethyl-, iso-Butyl- und tert.-Butyl-Gruppe sowie die Methoxygruppe.

Als Aralkylreste $R_1$ und $R_2$ kommen solche in Frage, deren gerad kettiger oder verzweigter Alkylenanteil 1-5 Kohlenstoffatome enthält. Bevorzugte Aralkylreste sind der Benzyl-, Phenethyl-der 4-Chlorphenethyl-Rest.

Unter Aralkenylreste $R_1$ sind solche zu verstehen, deren Alkenylenanteil 2-3 Kohlenstoffatome enthält. Hier sind bevorzugt der Styrylrest und der 4-Chlor-styryl-Rest.

Unter Halogen ist in allen Fällen Fluor, Chlor und Brom zu verstehen.

Als Alkylgruppen $R_2$ kommen geradkettige oder verzweigte mit 1-16 C-Atomen in Frage, bevorzugt ist die Methyl- und Octylgruppe.

Die Acylreste $R_2$ leiten sich von aliphatischen Carbonsäuren mit 2-16 C-Atomen, von araliphatischen und von aromatischen Carbonsäuren ab. Bevorzugte Acylgruppen sind Acetyl, Isobutyroyl, Cinnamoyl, Benzoyl, 4-Chlor-benzoyl und 4-Aminobenzoyl sowie n-Octanoyl und n-Hexadecanoyl.

Die niederen Alkylgruppen $R_3$ und $R_4$ können geradkettig oder verzweigt sein und enthalten 1-6, vorzugsweise 1-4 Kohlenstoffatome, wobei die Methyl- und Ethylgruppen bevorzugt sind.

n bedeutet bevorzugt die Zahl 2.

Als Ester der Carbonsäuren der allgemeinen Formel I kommen solche mit niederen einwertigen Alkoholen (wie z.B. Methanol oder Ethanol) oder mit mehrwertigen Alkoholen (wie z.B. Glycerin) in Frage, es seien aber auch solche Alkohole eingeschlossen, die noch andere funktionelle Gruppe enthalten, wie z.B. Ethanolamin.

Die erfindungsgemäßen, von den Carbonsäuren der allgemeinen Formel I abgeleiteten Amide enthalten als Aminkomponente z.B. Ammoniak, p-Aminobenzoesäure, β-Alanin, Ethanolamin und 2-Amino-propanol, wobei die bis hierher genannten bevorzugt sein sollen. Es kommen aber auch Alkylamine wie z.B. Isopropylamin oder tert.-Butylamin, Dialkylamine wie Diethylamin sowie cyclische Amine wie z.B. Morpholin oder 4-Alkyl-bzw. -Aralkyl- bzw. -Arylpiperazine, z.B. 4-Methylpiperazin, 4-(4-Chlorbenzyl)-piperazin oder 4-(3-Methoxy-phenyl)-piperazin in Frage.

Die obige Definition der erfindungsgemäßen Verbindungen soll auch alle möglichen Stereoisomeren sowie ihre Mischungen umfassen.

Die Herstellung der Verbindungen der allgemeinen Formel I ist dadurch gekennzeichnet, daß man ein Amin der allgemeinen Formel II

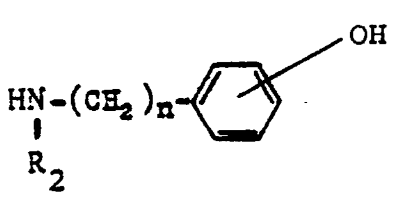

(II),

in welcher $R_2$ und n die oben angegebene Bedeutung haben,
gegebenenfalls unter intermediärem Schutz der Amino-bzw. Hydroxylgruppe in an sich bekannter Weise in beliebiger Reihenfolge mit einer Sulfonsäure der allgemeinen Formel III

$$R_1\text{-}SO_2OH \qquad (III),$$

in welcher $R_1$ die oben angegebene Bedeutung hat,
bzw. einem Derivat derselben und mit einer Verbindung der allgemeinen Formel IV

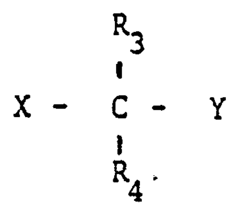

(IV),

umsetzt.

In Formel IV haben $R_3$ und $R_4$ die oben angegebene Bedeutung, X stellt eine reaktive Gruppe dar, und unter Y ist die Gruppe $-COOR_5$ (wobei $R_5$ ein Wasserstoffatom oder eine niedere Alkylgruppe darstellt) oder eine Säureamidgruppe zu verstehen. Y kann aber auch einen Rest darstellen, der nach erfolgter Kondensation in eine Säureamidgruppe oder in die $-COOR_5$-Gruppe überführt wird, worauf man gegebenenfalls einen bestimmten Substituenten $R_5$ anschließend an die Kondensation in an sich bekannter Weise in einen anderen Substituenten $R_5$ umwandelt und die erhaltenen Verbindungen in pharmakologisch unbedenkliche Salze, Ester oder Amide überführt.

Für den Fall, daß $R_3$ und $R_4$ niedere Alkylgruppen bedeuten, können die Phenole der allgemeinen Formel II oder deren Reaktionsprodukte mit einer Sulfonsäure der allgemeinen Formel III auch mit einem Gemisch aus einem aliphatischen Keton, Chloroform und einem Alkalihydroxid umgesetzt werden. Vorzugsweise wird diese Variante zur Hetstellung von Isobuttersäure-Derivaten verwendet, wobei als Keton Aceton eingesetzt wird (vgl. hierzu Gazz.Chim.ital. 77 (1947), S. 431).

Das erfindungsgemäße Verfahren führt man zweckmäßig in zwei Stufen durch. Die Kondensation der Verbindungen der allgemeinen Formel II mit Sulfonsäuren III oder deren Derivaten einerseits und Verbindungen der allgemeinen Formel IV andererseits wird vorzugsweise so durchgeführt, daß man zunächst eine der beiden reaktiven Gruppen der Verbindung II mit einer leicht abspaltbaren Schutzgruppe blockiert, die erhaltene Verbindung mit einer Sulfonsäure III oder einem Derivat davon bzw. mit einer Verbindung der allgemeinen Formel IV umsetzt, die Schutzgruppe wieder abspaltet und anschließend dieses reaktive Zwischenprodukt mit der noch nicht eingesetzten Verbindung der allgemeinen Formel IV bzw. III umsetzt. Bevorzugt wird ein Verfahrensweg, bei dem die an der Aminogruppe geschützte Verbindung II zunächst mit einer Verbindung IV zur Umsetzung gebracht wird. Nach Abspaltung der Schutzgruppe erfolgt dann die Reaktion mit einer Sulfonsäure III bzw. mit einem ihrer Derivate.

Anstelle der freien Amine II kann man auch deren Salze einsetzen.

Eine weitere Möglichkeit zur Herstellung von Verbindungen der allgemeinen Formel I, ihrer Salze sowie ihrer Ester und Amide besteht darin, daß man ein Sulfonamid der allgemeinen Formel V

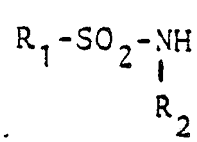

(V),

mit einer Verbindung der allgemeinen Formel VI

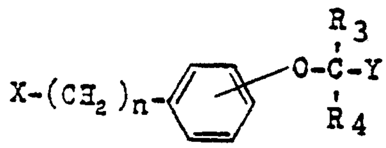

(VI)

zu der Verbindung VII

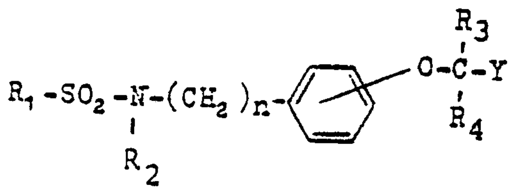

(VII),

umsetzt.

Die in den Formeln V, VI und VII verwendeten Symbole $R_1$, $R_2$, X, n, $R_3$, $R_4$ und Y haben die oben angegebene Bedeutung.

Eine dritte Synthesemöglichkeit besteht in einer Umacylierung:
Setzt man eine freie Sulfonsäure III mit einer Verbindung der allgemeinen Formel VIII

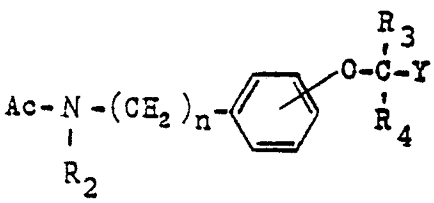

(VIII),

in welcher n, $R_2$ $R_3$, $R_4$ und Y die oben angegebene Bedeutung haben und Ac einen leicht austauschbaren Acylrest darstellt, in einem geeigneten Lösungsmittel um, so erhält man ebenfalls ein Sulfonamid der allgemeinen Formel VII.

Für den Fall, daß $R_2$ einen Acylrest darstellt, kommt bevorzugt eine vierte Synthesemöglichkeit in Frage:
Man setzt eine Verbindung der allgemeinen Formel I, in welcher $R_2$ ein Wasserstoffatom darstellt, mit einem reaktiven Derivat einer Carbonsäure, z.B. mit einem Säurechlorid, in einem geeigneten Lösungsmittel um. Bevorzugt ist dabei eine Verfahrensweise, bei der die Verbindung I (mit $R_2$ = H) in Form eines Esters der Phenoxycarbonsäure zur Umsetzung gebracht wird.

Als reaktive Derivate der Sulfonsäuren III kommen insbesondere die Halogenide sowie die Ester infrage. Die Umsetzungen der Sulfonsäurehalogenide mit Verbindungen der allgemeinen Formel II erfolgen zweckmäßig unter Zusatz eines säurebindenden Mittels, wie z.B. Alkaliacetat, Natriumhydrogencarbonat, Natriumcarbonat, Natriumphosphat, Calciumoxid, Calciumcarbonat oder Magnesiumcarbonat. Diese Funktion können aber auch organische Basen wie z.B. Pyridin oder Triethylamin übernehmen, wobei als inertes Lösungsmittel z.B. Ether, Benzol, Methylenchlorid, Dioxan oder ein Überschuß des tertiären Amins dient.

Bei Einsatz anorganischer Säurebinder verwendet man als Reaktionsmedium z.B. Wasser, wäßriges Ethanol oder wäßriges Dioxan.

Für eine Umsetzung der Verbindung II mit der Verbindung IV hat es sich als vorteilhaft herausgestellt, zunächst die Aminogruppe der Verbindung II in eine geschützte Gruppe, z.B. die Phthalimidgruppe, zu überführen, die nach der Umsetzung z.B. mit Hydroxylamin in an sich bekannter Weise leicht wieder gespalten werden kann. Es können auch andere aus der Peptidchemie bekannte Gruppen zum Schutz der Aminogruppe eingeführt und nach der Umsetzung wieder abgespalten werden. Bevorzugt ist die Blockierung der Aminogruppe durch eine Acylgruppe, wie die Formyl- oder Acetylgruppe, die nach der Umsetzung

mit starken Basen, wie z.B. Natriumhydroxid oder Kaliumhydroxid, aber auch mit wäßrigen Mineralsäuren,wie z.B. Salzsäure, leicht wieder abgespalten werden kann.

Als reaktive Verbindung IV kommt insbesondere diejenige infrage, bei der X das Anion einer starken Säure, z.B. einer Halogenwasserstoff- oder Sulfonsäure darstellt.

Die Reaktion kann weiterhin begünstigt werden, indem man die phenolische Hydroxylgruppe der Verbindung II z.B. durch Umsetzung mit Natriumalkoholat in ein Phenolat überführt. Die Reaktion der beiden Komponenten wird in Lösungsmitteln, wie z.B. Toluol oder Xylol, Methylethylketon oder Dimethylformamid, vorzugsweise in der Wärme durchgeführt.

Zur Alkylierung der Sulfonsäureamide V verwendet man bevorzugt Verbindungen VI, in denen X eine Arylsulfonyloxygruppe darstellt. Bevorzugt soll X eine Toluolsulfonyloxygruppe bedeuten. Als Alkylierungsmittel dienen also bevorzugt Arylsulfonsäurealkylester, eine Methode, die in ihrer Anwendung auf Sulfonsäureamide z.B. bei Klamann et al., Monatshefte für Chemie Bd. 83 (1952), S. 871, beschrieben ist. Die Umsetzungen erfolgen in alkalischem Milieu, bevorzugt ist als Reaktionsmedium heiße, konzentrierte Sodalösung.

Die Umacylierungsreaktion zwischen einer freien Sulfonsäure III und einem Acylamin VIII wird unter Einsatz bevorzugt äquimolarer Mengen beider Reaktionspartner in einem polaren Lösungmittel vorgenommen. Als solche polaren Lösungsmittel dienen z.B. Alkohole, insbesondere Ethanol und Methanol. Die Umsetzung verläuft bevorzugt bei der Siedetemperatur dieser Lösungsmittel. Als leicht austauschbarer Acylrest ist z.B. der Acetylrest zu nennen.

Als Substituenten Y der allgemeinen Formel IV, die in die -COOR$_5$-Gruppe überführt werden können, kommen beispielsweise die Nitril-, Carbaldehyd-, Hydroxymethyl-, Aminomethyl- und Formylgruppe infrage.

Die gegebenenfalls nachträgliche N-Alkylierung einer Verbindung der allgemeinen Formel I, in denen R$_2$ = Wasserstoff bedeutet, kann nach bekannten Methoden durchgeführt werden, vorzugsweise indem man eine Verbindung mit R$_2$ = Wasserstoff mit einem Alkylhalogenid oder einem Dialkylsulfat in Gegenwart eines säurebindenden Mittels, wie z.B. Natriumhydroxid, umsetzt.

Die Einführung einer Acylgruppe R$_2$ in ein Sulfonamid der allgemeinen Formel I (R$_2$ = H) erfolgt unter Bedingungen, wie sie für die Acylierung von Aminen üblich sind: Umsetzen mit einem aktiven Carbonsäure-Derivat, z.B. einem Säurehalogenid, einem gemischten Anhydrid oder einem aktiven Ester, in einem inerten Lösungsmittel in Gegenwart von Basen. Als inerte Lösungsmittel kommen beispielsweise Methylenchlorid, Benzol, Dimethylformamid u.ä. in Frage.

Die gegebenenfalls im Anschluß an die Kondensation durchzuführende Umwandlung des Substituenten R$_5$ erfolgt beispielsweise durch Verseifung der Carbonsäureester (R$_5$ = Alkyl) zu den entsprechenden Carbonsäuren (R$_5$ = Wasserstoff) mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium-oder Kaliumhydroxid) in einem gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest R$_5$ durch Umestern in einen Ester mit einem anderen Rest R$_5$ umwandeln. Die Veresterung der Carbonsäuren wird zweckmäßig in Gegenwart eines sauren Katalysators, wie z.B. Chlorwasserstoff, Schwefelsäure, p-Toluolsulfonsäure oder eines stark sauren Ionenaustauschharzes, vorgenommen. Umesterungen hingegen erfordern den Zusatz einer geringen Menge einer basischen Substanz, z.B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z.B. Glykol, oder Alkohole mit anderen funktionellen Gruppen, wie Ethanolamin oder Glykolether.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminokomponenten kommen z.B. Ammoniak, Alkylamine, Dialkylamine, aber auch Aminoalkohole wie z.B. Ethanolamin und 2-Aminopropanol sowie Aminosäuren wie z.B. p-Aminobenzoesäure, $\beta$-Alanin und andere infrage. Andere wertvolle Aminkomponenten sind Alkyl-, Aralkyl- und Arylpiperazine.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks-und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dasis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendung pro Tag wirksam, um die gewünschten Resultate zu erhalten.

Die nachfolgenden Beispiele zeigen einige der zahlreichen Verfahrensvarianten, die zur Synthese der erfindungsgemäßen Verbindungen verwendet werden können. Sie sollen jedoch keine Einschränkung des Erfindungsgegenstandes darstellen.

Beispiel 1

3-[2-(4-Chlor-phenylsulfonylamino)ethyl]phenoxyessigsaeure

a) 3-[2-(Acetamino)ethyl]phenol

Zu einer Loesung aus 27.2 g (157 mmol) 3-Hydroxy-phenethylamin, 280 ml Methylenchlorid und 47.6 g (470 mmol) Triethylamin tropft man unter Kuehlen. (Eisbad) eine Loesung aus 24.6 g (313 mmol) Acetylchlorid und 30 ml Methylenchlorid. Dann wird eine Stunde bei Raumtemperatur geruehrt, mit 2 N-HCl, Wasser und $NaHCO_3$-Loesung extrahiert, mit Natriumsulfat getrocknet und eingedampft. Der oelige Rueckstand wird mit 156 ml 2 N NaOH und 320 ml Methanol vermischt und das Gemisch unter Stickstoff zwei Stunden auf 40 °C erwaermt. Dann destilliert man Methanol ab, behandelt mit Aktivkohle und saeuert in der Kaelte mit konz. Salzsaeure an. Man extrahiert nun das Produkt mit Methylenchlorid, dem etwas Aceton zugesetzt wurde, trocknet die Methylenchloridphase mit $Na_2SO_4$ und dampft ein. Das oelige Rohprodukt (Ausb. 23.3 g, 83 % d.Th.) wird ohne weitere Reinigung weiterverarbeitet.

b) 3-[2-(Acetamino)ethyl]phenoxyessigsaeure-ethylester

Ein Gemisch aus 23.3 g (130 mmol) 3-[2-(Acetamino)ethyl]phenol, 230 ml Butanon, 26.1 g (156 mmol) Bromessigsaeure-ethylester und 25.7 g (186 mmol) pulv. $K_2CO_3$ sicc. wird 8 Stunden auf Rueckflußtemperatur gehalten, dann saugt man heiß ab und waescht mit Butanon nach. Nach dem Eindampfen kristallisiert das Produkt aus. Man verreibt mit Ligroin, saugt ab und reinigt durch Digerieren mit Ligroin. Ausb. 30.7 g (89 % d.Th.), Schmp. 82-83 °C.

c) 3-(2-Aminoethyl)phenoxyessigsaeure

Ein Gemisch aus 23.6 g (89 mmol) 3-[2-(Acetamino)ethyl]phenoxyessigsaeureethylester und 200 ml 3 N-HCl wird 6 Stunden auf Rueckflußtemperatur gehalten, dann gibt man Aktivkohle zu und saugt heiß ab. Nach Zugabe von 50 ml conc. HCl laeßt man laengere Zeit in der Kaelte stehn, saugt ab. waescht mit kalter 2N-HCl und trocknet ueber Kaliumhydroxid. Ausbeute 12.0 g (58 % d.Th.) Hydrochlorid, Schmp. 216-217 °C.

d) 3-(2-Aminoethyl)phenoxyessigsaeure-ethylester

Ein Gemisch aus 10.7 g (46 mmol) 3-(2-Aminoethyl)phenoxyessigsaeure, 100 ml absolutem Ethanol und einer Spatelspitze p-Toluolsulfonsaeure wird drei Stunden auf Rueckflußtemperatur gehalten, dann kuehlt man stark ab und saugt die ausgefallenen Kristalle ab. Nach Umkristallisieren aus Ethanol erhaelt man 8.3 g (69 % d.Th.) Hydrochlorid mit dem Schmp. 119-120 °C.

e) 3-[2-(4-Chlor-phenylsulfonylamino)ethyl]phenoxyessigsaeure-ethylester

Zu einem Gemisch aus 8.3 g (32 mmol) 3-(2-Aminoethyl)phenoxyessigsaeureethylester-hydrochlorid und 8.0 g (66 mmol) Triethylamin gibt man bei 0 °C unter Ruehren 6.6 g (32 mmol) 4-Chlor-benzolsulfochlorid zu und ruehrt weitere drei Stunden bei 0 °C, anschließend 4 Stunden bei Raumtemperatur. Danach wird mit verd. Salzsaeure und Wasser extrahiert, mit Natriumsulfat getrocknet und schließlich eingedampft. Nach Umkristallisieren aus einem Toluol-Heptan-Gemisch erhaelt man 10.4 g (82 % d.Th.) Substanz mit dem Schmp. 65-66° C.

f) 3-[2-(4-Chlor-phenylsulfonylamino)ethyl]phenoxyessigsaeure

Ein Gemisch aus 6.2 g (15.6 mmol) Ethylester, 40 ml 2 N-NaOH und 40 ml Ethanol wird drei Stunden bei 60° C geruehrt, dann dampft man das Ethanol im Vak. ab, extrahiert die alkalische Loesung mit Ether und faellt anschließend durch Zugabe von conc. HCl die Saeure aus. Man saugt sie ab. waescht mit Wasser und kristallisiert aus 50proz. waeßrigem Ethanol um. Ausbeute 5.1 g (88 % d.Th.), Schmp. 119-120° C.

In analoger Weise erhaelt man aus 3-(2-Aminoethyl)phenoxyessigsaeure-ethylester-hyrochlorid und

b) 4-Cynao-benzolsulfonylchlorid:

3-[2-(4-Cyano-phenylsulfonylamino)ethyl]phenoxyessigsaeure-ethylester

Rohausb.: quantitativ, farbl. Oel.

und daraus

3-[2-(4-Cyano-phenylsulfonylamino)ethyl]phenoxyessigsaeure

Ausb. 81 % d.Th., Schmp. 136-137° C (waeßr. Ethanol)

f) 4-(4-Chlorphenyl)-benzolsulfochlorid:

3-[2-(4-(4-Chlorphenyl)phenylsulfonylamino)ethyl]phenoxyessigsäure-ethylester

Ausb. 87 % d.Th., Schmp. 93-94° C (Ethanol)

und daraus

3-[2-(4-(4-Chlorphenyl)phenylsulfonylamino)ethyl]phenoxyessigsäure

Ausb. 86 % d.Th., Schmp. 147-148° C (Eisessig).

Referenz-Beispiel 2

3-[2-(Phenylsulfonylamino)ethyl]phenoxyessigsaeure

Man erwaermt ein Gemisch aus 11.6 g (50 mmol) 3-(2-Aminoethyl)phenoxyessigsaeure-hydrochlorid, 200 ml Wasser und 60 mmol Kaliumcarbonat auf 80° C und gibt bei dieser Temperatur langsam und unter Ruehren 8.83 g (50 mmol) Benzolsulfochlorid zu. Danach wird weitere zwei Stunden bei 80° C geruehrt, dann kuehlt man ab und bringt mittels Salzsaeure auf pH 2. Nach Stehen in der Kaelte wird abgesaugt, mit eiskaltem Wasser gewaschen, getrocknet und aus einem Essigester-Ligroin-Gemisch umkristallisiert.

Ausb. 12.4 g (75 % d.Th.), Schmp. 78-79° C.

Beispiel 3

2-[2-(4-Chlor-phenylsulfonylamino)ethyl]phenoxyessigsaeure

a) 2-[2-(Acetamino)ethyl]phenol

Man versetzt eine Loesung aus 58 g (0.214 mmol) o-Hydroxyphenethylamin-hydrobromid und 500 ml trocknem Pyridin unter Ruehren und Eiskuehlung tropfenweise mit 37 g (0.47 mol) Acetylchlorid. Dann ruehrt man noch drei Stunden nach und gießt auf Eis. Das Gemisch wird mit conc. Ammoniak alkalisch gestellt und mit Methylenchlorid extrahiert. Die vereinigten organischen Extrakte werden getrocknet und eingedampft. Den Rueckstand verreibt man mit Ligroin.

Ausb. 30.9 g (81 % d.Th.), Schmp. 133-136° C.

b) 2-[2-(Acetamino)ethyl]phenoxyessigsaeure-ethylester

24.7 g (138 mmol) 2-[2-(Acetamino)ethyl]phenol werden mit 38.1 g (0.276 mol) Kaliumcarbonat, 34.6 g (0.207 mol) Bromessigsaeureethylester und 500 ml Butanon unter Ruehren 24 Stunden auf Rueckflußtemperatur erhitzt. Dann wird der organische Niederschlag abfiltriert und das Filtrat eingedampft. Den Rueckstand nimmt man in Methylenchlorid auf, waescht die Loesung mit 2 N Natronlauge, trocknet und dampft ein.

Ausb. 31.7 g (87 % d.Th.), farbloses Oel.

c) 2-(2-Aminoethyl)phenoxyessigsaeure

Ein Gemisch aus 39.5 g (148 mmol) 2-[2-(Acetamino)ethyl]phenoxyessigsaeure-ethylester, 82.9 g (1.48 mol) Aetzkali, 195 ml Ethanol und 160 ml Wasser werden unter Ruehren 48 Stunden auf Rueckflußtemperatur erhitzt. Nach dem Erkalten wird mit conc. Salzsaeure auf pH 5 gestellt und das ausgefallene Kaliumchlorid abfiltriert. Das Filtrat wird eingedampft. Man erhaelt als Rueckstand 33.2 g Hydrochlorid als Rohprodukt, das etwas Kaliumchlorid enthaelt.

d) 2-(2-Aminoethyl)phenoxyessigsaeure-ethylester

23.4 g des vorgenannten Rohproducts werden in 100 ml abs. Ethanol suspendiert und unter Eiskuehlung mit Chlorwasserstoff gesaettigt. Anschalieβend laesst man das Gemisch ueber Nacht bei Raumtemperatur stehen und erhitzt dann noch eine Stunde auf Rueckfluβtemperatur. Dann dampft man ein, nimmt den Rueckstand in Wasser auf und klaert die entstandene Loesung mit Kohle. Das Filtrat wird eingedampft und der wasserfreie Rueckstand mit abs. Ethanol ausgeruehrt. Man filtriert das unloesliche Kaliumchlorid ab und dampft das Filtrat ein. Der Rueckstand wird durch Verreiben mit einem Gemisch aus Ether und Aceton zur Kristallisation gebracht. Man erhaelt 23.6 g (87 % d.Th.) Hydrochlorid, Schmp. 76-79 °C.

e) 2-[2-(4-Chlor-phenylsulfonylamino)ethyl]phenoxyessigsaeure-ethylester

Man versetzt ein Gemisch aus 2.59 g (10 mmol) 2-(2-Aminoethyl)phenoxyessigsaeureethylester-hydrochlorid, 3.0 g (30 mmol) Triethylamin und 30 ml Methylenchlorid unter Ruehren bei 0 bis 5 °C mit 2.11 g (10 mmol) 4-Chlorbenzolsulfochlorid. Dann entfernt man das Kuehlbad und ruehrt noch 3 Stunden bei Raumtemperatur nach. Anschlieβend setzt man Kohle zu und filtriert das ausgefallene Triethylaminhydrochlorid ab. Das Filtrat wird zunaechst mit 0.5 N Salzsaeure, dann mit 0.5 N Natronlaufe und Wasser gewaschen, getrocknet und eingedampft. Man erhaelt als Rueckstand 4.0 g (quant. Ausb.) der gewuenschten Verbindung als farbloses Oel.

f) 2-[2-(4-Chlor-phenylsulfonylamino)ethyl]phenoxyessigsaeure

4.0 g (10 mmol) des nach e) erhaltenen Esters werden in 50 ml Methanol geloest und unter Ruehren mit 30 ml 1 N Kalilauge versetzt. Man ruehrt das Gemisch 16 Stunden bei 50 °C und destilliert dann im Vak. das Methanol ab. Die als Rueckstand erhaltene waeβr. Loesung wird zunaechst mit Ether gewaschen, dann mit Kohle geklaert und schlieβlich mit verduennter Salzsaeure angesaeuert. Das abgeschiedene Oel wird mit Methylenchlorid extrahiert. Die vereinigten Extrakte werden mit Kohle geklaert, getrocknet und eingedampft. Man erhaelt als Rueckstand ein farbloses Oel, das nach Anreiben mit Ligroin kristallisiert.

Ausb. 2.0 g (54 % d.Th.), Schmp. 113-115 °C.

In analoger Weise erhaelt man aus

2-(2-Aminoethyl)phenoxyessigsaeure-ethylester und

a) p-Toluolsulfonylchlorid:
2-[2-(4-Methyl-phenylsulfonylamino)ethyl]phenoxyessigsaeure-ethylester
Ausb. quantitativ, farbloses Oel.
und daraus
2-[2-(4-Methyl-phenylsulfonylamino)ethyl]phenoxyessigsaeure
Ausb. 51 % d.Th., Schmp. 120-122 °C.

b) 4-Methoxy-benzolsulfonylchlorid:
2-[2-(4-Methoxy-phenylsulfonylamino)ethyl]phenoxyessigsaeure-ethylester
Ausb. quantitativ, farbl. Oel.
und daraus
2-[2-(4-Methoxy-phenylsulfonylamino)ethyl]phenoxyessigsaeure
Aus. 56 % d.Th., Schmp. 131-133 °C.

Beispiel 4

2-[2-(Phenylsulfonylamino)ethyl]phenoxyessigsaeure

Man versetzt eine Loesung aus 17.5 g (68 mmol) 2-(2-Aminoethyl)phenoxyessigsaeureethylester-hydrochlorid und 200 ml Pyridin unter Ruehren bei 0 - 5 °C mit 12.5 g (71 mmol) Benzolsulfonylchlorid. Dann entfernt man das Kuehlbad und ruehrt zunaechst 4 Stunden bei Raumtemperatur, dann 6 Stunden bei 60 °C. Anschlieβend gieβt man auf Eis, saeuert das waeβrige Gemisch an und extrahiert mit Methylenchlorid. Die Extrakte werden getrocknet und eingedampft. Man erhaelt als Rueckstand 18.6 g (76 % d.Th.) 2-[2-(Phenylsulfonylamino)ethyl]phenoxyessigsaeure-ethylester als farbloses Oel.

Ein Gemisch aus 13.6 g (37 mmol) dieses Esters, 100 ml 1 N Kalilauge und 150 ml Methanol wird 16 Stunden bei 60 °C geruehrt. Dann destilliert man das Methanol im Vakuum ab und saeuert den waeβrigen Rueckstand an. Man erhaelt 8.6 g (69 % d.Th.) der Titelverbindung mit dem Schmp. 130-133 °C.

Beispiel 5

3-[2-(N-Methyl-3-chlor-phenylsulfonylamino)ethyl]phenoxyessigsäure

Man dampft ein Gemisch aus 8.0 g (20.1 mmol) 3-[2-(3-Chlor-phenylsulfonylamino)ethyl]-

phenoxyessigsäure-ethylester, 100 ml abs. Ethanol und 20.1 mmol Natriummethylat zur Trockne, löst den Rückstand in 50 ml abs. DMF und gibt 2.85 g (20.1 mmol) Methyliodid zu. Nach 3 Stdn. Rühren bei 80° C wird abgekült, in Wasser eingerührt und mit Methylenchlorid extrahiert. Man trocknet die organische Phase mit $Na_2SO_4$, dampft ein und chrcmatografiert an einer Kieselgel-Säule mittels Methylenchlorid-Methanol (99:1 Vol.). Ausb. 7.5 g (91 % d.Th.) 3-[2-(N-Methyl-3-chlor-phenylsulfonylamino)ethyl]phenoxyessigsäure-ethylester. Farbloses Öl.

Die Verseifung dieses Esters zur Carbonsäure erfolgt in Analogie zu Beispiel 1 f.
Ausb. 90 % d.Th., Schmp. 138-139° C (Ethanol + Wasser 2:1)

In analoger Weise erhält man aus
3-[2-(3-Chlor-phenylsulfonylamino)ethyl]phenoxyessigsäure-ethylester und
Benzylchlorid:

a) 3-[2-(N-Benzyl-3-chlor-phenylsulfonylamino)ethyl]phenoxyessigssäure-ethylester
Ausb. 90 % d.Th., farbloses Öl
und daraus
3-[2-(N-Benzyl-3-chlor-phenylsulfonylamino)ethyl]phenoxyessigsäure
Ausb. 85 % d.Th., Schmp. 134-135° C (Ethanol + Wasser 2:1 Vol)
und aus
3-[2-(4-Chlor-phenylsulfonylamino)ethyl]phenoxyessigsäure-ethylester und
n-Octylchlorid:

b) 3-[2-(N-(n-Octyl)-4-chlor-phenylsulfonylamino)ethyl]phenoxyessigsäure-ethylester
Ausb. 69 % d.Th., farbloses Öl
und daraus
3-[2-(N-(n-Octyl)-4-chlor-phenylsulfonylamino)ethyl]phenoxyessigsäure
Ausb. 98 % d.Th., Schmp. 68-69° C (Ethanol + Wasser 2:1 Vol)

Beispiel 6

3-[2-(N-Acetyl-3-chlor-phenylsulfonylamino)ethyl]phenoxyessigsäure-ethylester

Zu einem eiskalten Gemisch aus 8.0 g (20.1 mmol) 3-[2-(3-Chlor-phenylsulfonylamino)ethyl]-phenoxyessigsäure-ethylester, 100 ml Methylenchlorid und 3.1 g (30.2 mmol) Triethylamin tropft man innerhalb 15 min eine Lösung aus 1.58 g (20.1 mmol) Acetylchlorid und 50 ml Methylenchlorid. Man rührt dann zwei Stdn. bei Raumtemperatur, extrahiert anschließend mit 2 N HCl und Wasser und trocknet mittels $Na_2SO_4$. Nach Chromatographie an einer Kieselgel-Säule mittels Methylenchlorid erhält man 4.95 g (63 % d.Th.) Ester in Form eines farblosen Öls.

In analoger Weise erhält man aus
3-[2-(4-Chlor-phenylsulfonylamino)ethyl]phenoxyessigsäure-ethylester und

a) n-Octanoylchlorid:
3-[2-(N-Octanoyl)-4-chlor-phenylsulfonylamino)ethyl]phenoxyessigsäure-ethylester
Ausb. 67 % d.Th., farbloses Öl.

b) n-Hexadecanoylchlorid:
3-[2-(N-n-Hexacecanoyl)-4-chlor-phenylsulfonylamino)ethyl]phenoxyessigsäure-ethylester
Ausb. 71 % d.Th., farbloses Öl.

**Patentansprüche**

1. Phenoxyalkylcarbonsäure-Derivate der allgemeinen Formel I

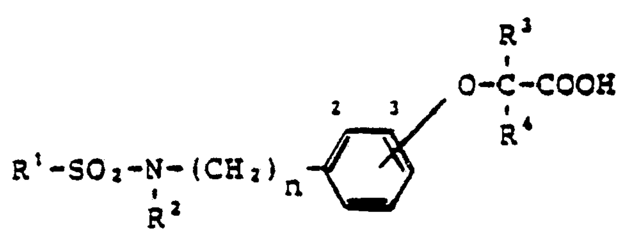

(I)

in welcher die Sulfonylaminoalkylgruppe in ortho- oder in meta-Stellung zur Phenoxyalkylcarbonsäure-Gruppe steht und

in welcher

| | |
|---|---|
| $R_1$ | eine Aryl-, Aralkyl- oder eine Aralkenylgruppe bedeutet, deren Arylrest jeweils gegebenenfalls ein- oder mehrfach durch Halogen, Cyan, Alkyl, Trifluormethyl, Alkoxy, Hydroxyl, Nitro, Amino, Alkylamino, Dialkylamino, Acylamino, Acyl, Azido oder gegebenenfalls durch Halogen, Cyan, Alkyl, Trifluormethyl oder Alkoxy substituiertes Phenyl substituiert sein kann, |
| $R_2$ | Wasserstoff, eine Alkyl-, Aralkyl- oder eine Acylgruppe darstellt, |
| $R_3$ und $R_4$, | die gleich oder verschieden sein können, Wasserstoff oder eine niedere Alkylgruppe und |
| n | die Zahlen 1 - 3 |

bedeuten, sowie deren physiologisch unbedenkliche Salze, Ester und Amide

mit der Maßgabe, daß die Phenoxyalkylcarbonsäure-Gruppe sowie deren physiologisch unbedenkliche Salze und $C_{1-4}$-Alkylester nicht in meta-Stellung stehen darf, wenn

$R_1$ Naphthyl, ß-Styryl, Phenyl$(CH_2)_p$, p die Zahl 0, 1, 2 oder 3, wobei Phenyl ein- oder mehrfach durch Halogen, $C_1$-$C_4$-Alkyl, $C_1$-$C_4$-Alkoxy, Hydroxyl, Trifluormethyl, Nitro oder Amino substituiert sein kann,
n die Zahl 1, 2 oder 3
$R_2$ Wasserstoff und $R_3$ und $R_4$ Wasserstoff

bedeuten,

jedoch 3-[2-(4-Chlor-phenylsulfonylamino)ethyl]phenoxyessigsäure sowie deren physiologisch unbedenkliche Salze, Ester und Amide nicht ausgenommen sind.

2. Phenoxyalkylcarbonsäure-Derivate der allgemeinen Formel I gemäß Anspruch 1, in der

| | |
|---|---|
| $R_1$ | eine Phenyl- oder Biphenylgruppe bedeutet, die jeweils gegebenenfalls durch Chlor, Brom, Cyan, Methyl, Trifluormethyl und Methoxy substituiert sein kann, |
| $R_2$ | , eine Methyl-, Octyl-, Benzyl-, Acetyl, n-Octanoyl- und n-Hexadecanoylgruppe darstellt, |
| $R_3$ und $R_4$ | Wasserstoff und |
| n | die Zahl 2 bedeuten, |

sowie deren physiologisch unbedenkliche Salze, Ester und Amide.

3. Verfahren zur Herstellung von Phenoxyalkylcarbonsäure-Derivaten der allgemeinen Formel I

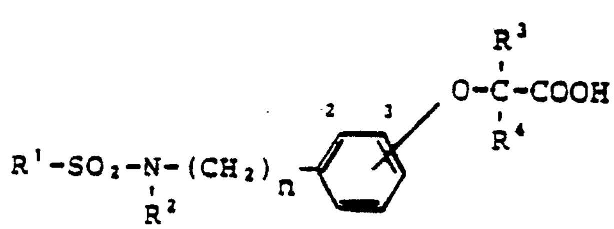

(I)

in welcher die Sulfonylaminoalkylgruppe in ortho- oder in meta-Stellung zur Phenoxyalkylcarbonsäure-Gruppe steht und

in welcher

| | |
|---|---|
| $R_1$ | eine Aryl-, Aralkyl- oder eine Aralkenylgruppe bedeutet, deren Arylrest jeweils gegebenenfalls ein- oder mehrfach durch Halogen, Cyan, Alkyl, Trifluormethyl, Alkoxy, Hydroxy, Nitro, Amino, Alkylamino, Dialkylamino, Acylamino, Acyl, Azido oder gegebenenfalls durch Halogen, Cyan, Alkyl, Trifluormethyl oder Alkoxy substituiertes Phenyl substituiert sein kann, |
| $R_2$ | Wasserstoff, eine Alkyl-, Aralkyl- oder eine Acylgruppe darstellt, |
| $R_3$ und $R_4$, | die gleich oder verschieden sein können, Wasserstoff oder eine niedere Alkylgruppe und |
| n | die Zahlen 1 - 3 |

bedeuten, sowie deren physiologisch unbedenkliche Salze, Ester und Amide
mit der Maßgabe, daß die Phenoxyalkylcarbonsäure-Gruppe sowie deren physiologisch unbedenkliche Salze und $C_{1-4}$-Alkylester nicht in meta-Stellung stehen darf, wenn

$R_1$ Naphthyl, ß-Styryl, Phenyl$(CH_2)_p$; p die Zahl 0, 1, 2 oder 3, wobei Phenyl ein- oder mehrfach durch Halogen, $C_1$-$C_4$-Alkyl, $C_1$-$C_4$-Alkoxy, Hydroxyl, Trifluormethyl, Nitro oder Amino substituiert sein kann,
n die Zahl 1, 2 oder 3
$R_2$ Wasserstoff und $R_3$ und $R_4$ Wasserstoff bedeuten, jedoch 3-[2-(4-Chlor-phenylsulfonylamino)ethyl]-phenoxyessigsäure sowie deren physiologisch unbedenkliche Salze, Ester und Amide nicht ausgenommen sind, dadurch gekennzeichnet, daß man
a) ein Amin der allgemeinen Formel II

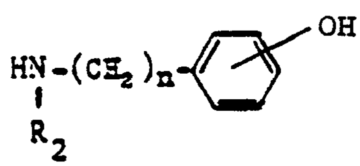 (II),

in welcher $R_2$ und n die oben angegebene Bedeutung haben,

gegebenenfalls unter intermediärem Schutz der Amino-bzw. Hydroxylgruppe in an sich bekannter Weise in beliebiger Reihenfolge mit einer Sulfonsäure der allgemeinen Formel III

$R_1$-$SO_2OH$ (III) ,

in welcher $R_1$ die oben angegebene Bedeutung hat,

bzw. einem Derivat derselben und mit einer Verbindung der allgemeinen Formel IV

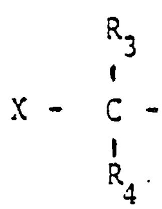 (IV),

umsetzt,
in der $R_3$ und $R_4$ die oben genannte Bedeutung haben, und X eine reaktive Gruppe darstellt und Y die Gruppe $-COOR_5$ bedeutet, in der $R_5$ Wasserstoff oder eine niedere Alkylgruppe ist, oder Y eine Säureamidogruppe oder einen Rest darstellt, der nach erfolgter Kondensation in eine Säureamidogruppe oder in die $COOR_5$-Gruppe überführt wird, oder
b) ein Sulfonamid der allgemeinen Formel V

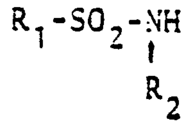

(V),

in der $R_1$ und $R_2$ die oben genannten Bedeutungen haben, mit einer Verbindung der allgemeinen Formel VI

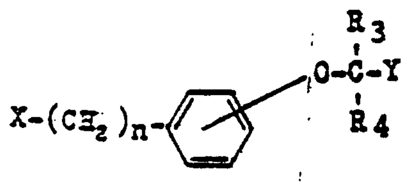

(VI)

in der n, X, Y, $R_3$ und $R_4$ die oben genannten Bedeutungen haben,
zu der Verbindung VII

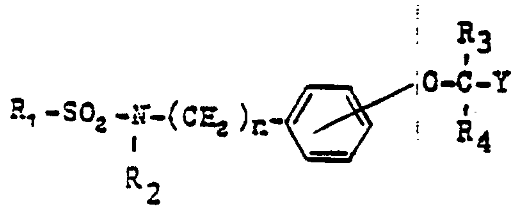

(VII),

in der n, $R_1$,$R_2$,$R_3$,$R_4$ und Y die oben genannten Bedeutungen haben,
umsetzt,
worauf man gegebenenfalls Verbindungen der allgemeinen Formel I mit R = Wasserstoff durch bekannte Verfahren N-alkyliert oder N-acyliert und gegebenenfalls die erhaltenen Säurederivate der allgemeinen Formel I in die freie Säure oder gewünschtenfalls die erhaltene freie Säure der allgemeinen Formel I verestert, in ein Amid oder in Physiologisch verträgliche Salze überführt.

**4.** Verfahren zur Herstellung von Phenoxyalkylcarbonsäure-Derivate der allgemeinen Formel I gemäß Anspruch 3, in der

| | |
|---|---|
| $R_1$ | eine Phenyl- oder Biphenylgruppe bedeutet, die jeweils gegebenenfalls durch Chlor, Brom, Cyan, Methyl, Trifluormethyl und Methoxy substituiert sein kann, |
| $R_2$ | , eine Methyl-, Octyl-, Benzyl-, Acetyl-, n-Octanoyl- und n-Hexadecanoylgruppe darstellt, |
| $R_3$ und $R_4$ | Wasserstoff und |
| n | die Zahl 2 bedeuten, |

sowie deren physiologisch unbedenkliche Salze, Ester und Amide.

**5.** Arzneimittel, enthaltend eine Verbindung gemäß Anspruch 1,2 oder 7 sowie die üblichen pharmakologisch unbedenklichen Träger- und Hilfsstoffe.

**6.** Verwendung der Verbindungen gemäß Anspruch 1,2 oder 7 zur Herstellung von Arzneimitteln mit thrombozytenaggregationshemmender und/oder lipidsenkender Wirkung.

**7.** 3-[2-(4-Chlor-phenylsulfonylamino)ethyl]phenoxyessigsäure sowie deren physiologisch unbedenkliche Salze, Ester und Amide gemäß Anspruch 1.

**8.** Verfahren gemäß Anspruch 3 zur Herstellung von 3-[2-(4-Chlor-phenylsulfonylamino)ethyl]-phenoxyessigsäure sowie deren physiologisch unbedenklichen Salzen, Estern und Amiden.

## Claims

1. Phenoxyalkylcarboxylic acid derivatives of the general formula I

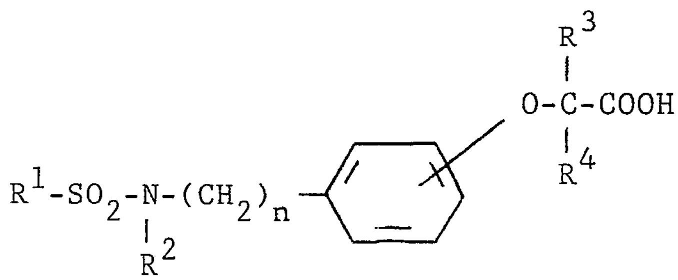

(I)

in which the sulphonylaminoalkyl group stands in the ortho- or meta-position to the phenoxyalkylcarboxylic acid group and in which $R^1$ signifies an aryl, aralkyl or an aralkenyl group, the aryl radical of which can, in each case, be possibly substituted one or more times by halogen, cyano, alkyl, trifluoromethyl, alkoxy, hydroxyl, nitro, amino, alkylamino, dialkylamino, acylamino, acyl, azido or phenyl possibly substituted by halogen, cyano, alkyl, trifluoromethyl or alkoxy, $R^2$ represents hydrogen, an alkyl, aralkyl or an acyl group, $R^3$ and $R^4$, which can be the same or different, hydrogen or a lower alkyl group and n the numbers 1 - 3, as well as their physiologically acceptable salts, esters and amides, with the proviso that the phenoxyalkylcarboxylic acid group, as well as its physiologically acceptable salts and $C_{1-4}$-alkylesters, must not stand in the meta-position when $R^1$ signifies naphthyl, ß-styryl, phenyl$(CH_2)_p$, p the numbers 0, 1, 2 or 3, whereby phenyl can be substituted one or more times by halogen, $C_1$-$C_4$-alkyl, $C_1$- $C_4$-alkoxy, hydroxyl, trifluoromethyl, nitro or amino, n the numbers 1, 2 or 3, $R^2$ hydrogen, and $R^3$ and $R^4$ hydrogen but 3-[2-(4-chlorophenylsulphonylamino)-ethyl]-phenoxyacetic acid, as well as its physiologically acceptable salts, esters and amides are not excluded.

2. Phenoxyalkylcarboxylic acid derivatives of the general formula I according to claim 1, in which $R^1$ signifies a phenyl or biphenyl group which, in each case, can possibly be substituted by chlorine, bromine, cyano, methyl, trifluoromethyl and methoxy, $R^2$ represents a methyl, octyl, benzyl, acetyl, n-octanoyl and n-hexadecanoyl group, $R^3$ and $R^4$ hydrogen and n the number 2, as well as their physiologically acceptable salts, esters and amides.

3. Process for the preparation of phenoxyalkylcarboxylic acid derivatives of the general formula I

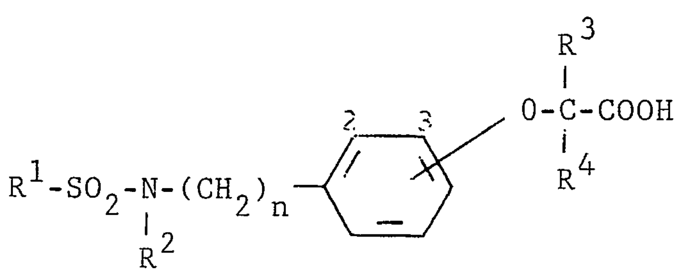

(I)

in which the sulphonylaminoalkyl group stands in the ortho- or meta-position to the phenoxyalkylcarboxylic acid group and in which $R^1$ signifies an aryl, aralkyl or an aralkenyl radical, the aryl radical of which can, in each case, be optionally substituted one or more times by halogen, cyano, alkyl, trifluoromethyl, alkoxy, hydroxyl, nitro, amino, alkylamino, dialkylamino, acylamino, acyl, azido or phenyl possibly substituted by halogen, cyano, alkyl, trifluoromethyl or alkoxy, $R^2$ represents hydrogen, an alkyl, aralkyl or an acyl group, $R^3$ and $R^4$, which can be the same or different, hydrogen or a lower alkyl group and n the numbers 1 - 3, as well as of their physiologically acceptable salts, esters and amides, with the proviso that the phenoxyalkylcarboxylic acid group, as well as its physiologically acceptable salts and $C_{1-4}$-alkylesters, must not stand in the meta-position when $R^1$ signifies naphthyl, ß-styryl, phenyl$(CH_2)_p$, p the numbers 0, 1, 2 or 3, whereby phenyl can be substituted one or more times by halogen, $C_1$-$C_4$-

alkyl, $C_1$-$C_4$-alkoxy, hydroxyl, trifluoromethyl, nitro or amino, n the number 1, 2 or 3, $R^2$ hydrogen and $R^3$ and $R^4$ hydrogen but 3-[2-(4-chlorophenylsulphonylamino)-ethyl]-phenoxyacetic acid, as well as its physiologically acceptable salts, esters and amides are not excluded, characterised in that one

a) reacts an amine of the general formula II

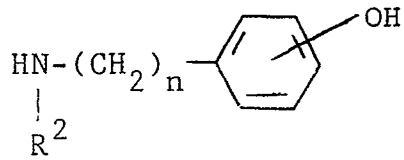

(II)

in which $R^2$ and n have the above-given meaning, possibly with intermediate protection of the amino or hydroxyl group, in per se known manner in any desired sequence with a sulphonic acid of the general formula III

$R^1$-$SO_2OH$ (III)

in which $R^1$ has the above-given meaning, or a derivative thereof and with a compound of the general formula IV

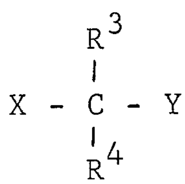

(IV)

in which $R^3$ and $R^4$ have the above-given meaning and X represents a reactive group and Y signifies the group -$COOR_5$, in which $R_5$ is hydrogen or a lower alkyl group, or Y represents an acid amido group or a radical which, after condensation has taken place, is converted into an acid amido group or into the -$COOR_5$ group, or

b) reacts a sulphonamide of the general formula V

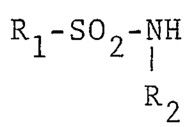

(V)

in which $R_1$ and $R_2$ have the above-given meaning, with a compound of the general formula VI

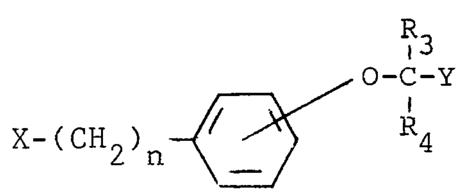

(VI)

in which n, X, Y, $R_3$ and $R_4$ have the above-given meanings, to give the compound VII

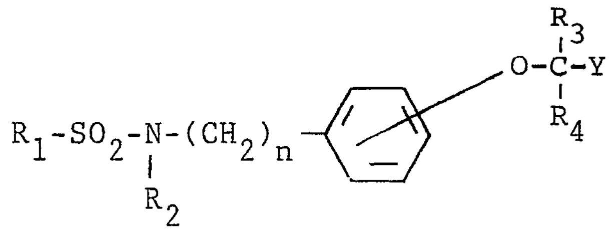

(VII)

in which n, $R_1$, $R_2$, $R_3$, $R_4$ and Y have the above-given meanings, whereupon one possibly N-alkylates or N-acylates compounds of the general formula I with R = hydrogen by known processes and possibly converts the acid derivatives obtained of the general formula I into the free acid or, if desired, esterifies the free acid obtained of the general formula I or converts into an amide or into physiologically acceptable salts.

4. Process for the preparation of phenoxyalkylcarboxylic acid derivatives of the general formula I according to claim 3, in which $R_1$ signifies a phenyl or biphenyl group which, in each case, can be substituted by chlorine, bromine, cyano, methyl, trifluoromethyl and methoxy, $R_2$ represents a methyl, octyl, benzyl, acetyl, n-octanoyl and n-hexadecanoyl group, $R_3$ and $R_4$ signifies hydrogen and n the number 2, as well as of their physiologically acceptable salts, esters and amides.

5. Medicaments containing a compound according to claim 1 or 2, as well as usual pharmacologically acceptable carrier and adjuvant materials.

6. Use of compounds according to claim 1 or 2 for the preparation of medicaments with thrombocyte aggregation-inhibiting and/or lipid-lowering action.

7. 3-[2-(4-Chlorophenylsulphonylamino)-ethyl]-phenoxyacetic acid, as well as its physiologically acceptable salts, esters and amides according to claim 1.

8. Process according to claim 3 for the preparation of 3-[2-(4-chlorophenylsulphonylamino)-ethyl]-phenoxyacetic acid, as well as of its physiologically acceptable salts, esters and amides.

**Revendications**

1. Dérivés d'acides phénoxyalkylcarboxyliques de formule générale I

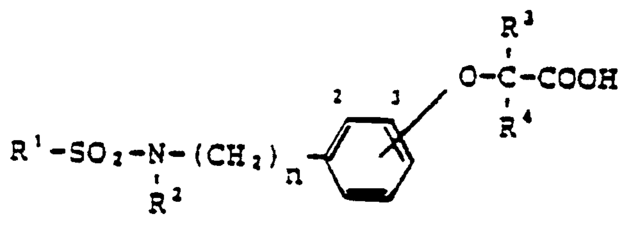

(I)

dans laquelle le groupe sulfonylaminoalkyle est en position ortho ou méta par rapport au groupe phénoxyalkylaminocarboxylique et dans laquelle

| | |
|---|---|
| $R_1$ | représente un groupe aryle, aralkyle ou aralcényle, dont le reste aryle peut être éventuellement une ou plusieurs fois substitué par un halogène, un groupe cyano, alkyle, trifluorométhyle, alcoxy, hydroxyle, nitro, amino, alkylamino, dialkylamino, acylamino, acyle, azido ou par un groupe phényle éventuellement substitué par un halogène, un groupe cyano, alkyle, trifluorométhyle ou alcoxy, |
| $R_2$ | représente l'hydrogène, un groupe alkyle, aralkyle ou acyle, |
| $R_3$ et $R_4$, | qui peuvent être identiques ou différents, représentent l'hydrogène ou un groupe alkyle inférieur, et |
| n | vaut 1 - 3, |

ainsi que leurs sels, esters et amides physiologiquement acceptables,
étant entendu que le groupe phénoxyalkylcarboxylique, ainsi que ses sels et esters alkyliques en $C_{1-4}$ physiologiquement acceptables ne se trouvent pas en position méta, lorsque

$R_1$ représente un groupe naphtyle, ß-styryle, phényl$(CH_2)_p$, p vaut 0, 1, 2 ou 3, le groupe phényle pouvant être substitué une ou plusieurs fois par un halogène, un groupe alkyle en $C_1$-$C_4$, alcoxy en $C_1$-$C_4$, hydroxyle, trifluorométhyle, nitro ou amino,
n vaut 1, 2 ou 3,
$R_2$ représente l'hydrogène, et $R_3$ et $R_4$ représentent l'hydrogène,

l'acide 3-[2-(4-chlorophénylsulfonylamino)éthyl]phénoxyacétique ainsi que ses sels, esters et amides physiologiquement acceptables n'étant toutefois pas exclus.

2. Dérivés d'acides phénoxyalkylcarboxyliques de formule générale I selon la revendication 1, où

$R_1$ représente un groupe phényle ou biphényle, qui peut être éventuellement substitué par un atome de chlore, brome, un groupe cyano, méthyle, trifluorométhyle et méthoxy,
$R_2$ représente un groupe méthyle, octyle, benzyle, acétyle, n-octanoyle et n-hexadécanoyle,
$R_3$ et $R_4$ représentent l'hydrogène, et
n vaut 2,

ainsi que leurs sels, esters et amides physiologiquement acceptables.

3. Procédé de préparation de dérivés d'acides phénoxyalkylcarboxyliques de formule générale I

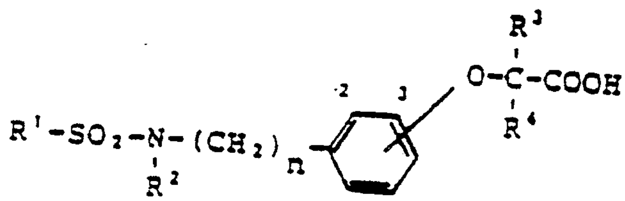

(I)

dans laquelle le groupe sulfonylaminoalkyle est en position ortho ou méta par rapport au groupe phénoxyalkylcarboxylique, et dans laquelle

$R_1$ représente un groupe aryle, aralkyle ou aralcényle, dont le reste aryle peut être éventuellement une ou plusieurs fois substitué par un halogène, un groupe cyano, alkyle, trifluorométhyle, alcoxy, hydroxyle, nitro, amino, alkylamino, dialkylamino, acylamino, acyle, azido ou par un groupe phényle éventuellement substitué par un halogène, un groupe cyano, alkyle, trifluorométhyle ou alcoxy,
$R_2$ représente l'hydrogène, un groupe alkyle, aralkyle ou acyle,
$R_3$ et $R_4$, qui peuvent être identiques ou différents, représentent l'hydrogène ou un groupe alkyle inférieur, et
n vaut 1 - 3,

ainsi que leurs sels, esters et amides physiologiquement acceptables,
étant entendu que le groupe phénoxyalkylcarboxylique, ainsi que ses sels et esters alkyliques en $C_{1-4}$ physiologiquement acceptables ne se trouvent pas en position méta, lorsque

$R_1$ représente un groupe naphtyle, ß-styryle, phényl$(CH_2)_p$, p vaut 0, 1, 2 ou 3, le groupe phényle pouvant être substitué une ou plusieurs fois par un halogène, un groupe alkyle en $C_1$-$C_4$, alcoxy en $C_1$-$C_4$, hydroxyle, trifluorométhyle, nitro ou amino,
n vaut 1, 2 ou 3,
$R_2$ représente l'hydrogène, et $R_3$ et $R_4$ représentent l'hydrogène,

l'acide 3-[2-(4-chlorophénylsulfonylamino)éthyl]phénoxyacétique ainsi que ses sels, esters et amides physiologiquement acceptables n'étant toutefois pas exclus,
caractérisé en ce que

a) on fait réagir une amine de formule générale II

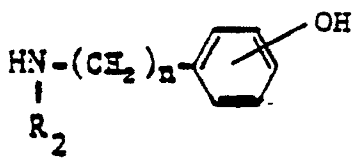 (II),

dans laquelle $R_2$ et n ont les significations mentionnées ci-dessus, éventuellement après protection provisoire des groupes amino ou hydroxyle, de manière connue en soi, dans un ordre quelconque, avec un acide sulfonique de formule générale III

$R_1$-$SO_2OH$ (III) ,

dans laquelle $R_1$ a la signification mentionnée ci-dessus, ou avec un de ses dérivés, et avec un composé de formule générale IV

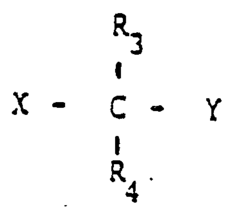 (IV) ,

dans laquelle $R_3$ et $R_4$ ont les significations mentionnées ci-dessus, et X représente un groupe réactif et Y le groupe -$COOR_5$, dans lequel $R_5$ représente l'hydrogène ou un groupe alkyle inférieur, ou Y représente un groupe amidoacide ou un groupe qui est transformé, après la condensation, en un groupe amidoacide ou en le groupe -$COOR_5$, ou

b) on fait réagir un sulfonamide de formule générale V

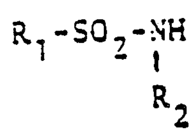 (V) ,

dans laquelle $R_1$ et $R_2$ ont les significations mentionnées ci-dessus, avec un composé de formule générale VI

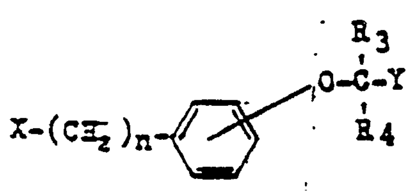 (VI)

dans laquelle n, X, Y, $R_3$ et $R_4$ ont les significations mentionnées ci-dessus, pour donner le composé VII

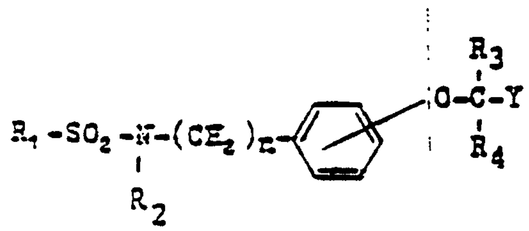

(VII) ,

dans laquelle n, $R_1$, $R_2$, $R_3$, $R_4$ et Y ont les significations mentionnées ci-dessus, après quoi éventuellement on effectue une N-alkylation ou une N-acylation des composés de formule générale I par des procédés connus, et l'on transforme éventuellement le dérivé d'acide de formule générale I obtenu en l'acide libre, ou éventuellement, on estérifie l'acide libre de formule générale I obtenu ou on le transforme en un amide ou un sel physiologiquement acceptable.

4. Procédé de préparation de dérivés d'acides phénoxyalkylcarboxyliques de formule générale I selon la revendication 3, dans lequel
   - $R_1$ représente un groupe phényle ou biphényle, qui peut être éventuellement substitué par un atome de chlore, brome, un groupe cyano, méthyle, trifluorométhyle et méthoxy,
   - $R_2$ représente un groupe méthyle, octyle, benzyle, acétyle, n-octanoyle et n-hexadécanoyle,
   - $R_3$ et $R_4$ représentent l'hydrogène, et
   - n vaut 2,

   ainsi que leurs sels, esters et amides physiologiquement acceptables.

5. Médicament, contenant un composé selon la revendication 1 ou 2, ainsi que des véhicules et adjuvants usuels, pharmacologiquement acceptables.

6. Utilisation des composés selon la revendication 1 ou 2, pour la préparation de médicaments ayant une activité anti-aggrégation des thrombocytes et/ou abaissant le taux de lipides.

7. Acide 3-[2-(4-chlorophénylsulfonylamino)éthyl]phénoxyacétique ainsi que ses sels, esters et amides physiologiquement acceptables selon la revendication 1.

8. Procédé selon la revendication 3 pour la préparation de l'acide 3-[2-(4-chlorophénylsulfonylamino)-éthyl]phénoxy-acétique ainsi que de ses sels, esters et amides physiologiquement acceptables.